# EUROPEAN PATENT SPECIFICATION

(11) **EP 3 973 266 B1**
(45) Date of publication and mention of the grant of the patent: **14.02.2024**
(21) Application number: 20725707.2
(22) Date of filing: 13.05.2020
(51) Int. Cl.: G01N 11/00, G01N 33/34, G01N 35/02

(54) **AUTOMATED POLYMER ANALYZING SYSTEM AND ITS USE**
AUTOMATISIERTES POLYMERANALYSESYSTEM UND DESSEN VERWENDUNG
SYSTÈME D'ANALYSE AUTOMATIQUE DE POLYMÈRE ET SON UTILISATION

(30) Priority: 23.05.2019 EP 19176296
(43) Date of publication of application: 30.03.2022
(73) Proprietor: LENZING AKTIENGESELLSCHAFT, 4860 Lenzing (AT)
(72) Inventor: RAMSAUER, Christoph, 5360 St. Wolfgang (AT); YALDEZ, Rolf, 4860 Lenzing (AT); KEMPTNER, Franz, 4693 Desselbrunn (AT); LEITNER, Helmut, 4863 Seewalchen (AT)
(74) Representative: Global Patent Management Lenzing AG
(86) International application number: PCT/EP2020/063277
(87) International publication number: WO 2020/234071

(56) References cited:
- EP-A1- 0 700 719
- WO-A1-2013/156147
- JP-B2- 3 225 503
- US-B2- 7 153 391

## Description

This invention relates to an automated system for the determination of polymer properties, in particular the limiting viscosity number, and the use of this system for monitoring the processing of the respective polymer.

### Prior Art

An automatic laboratory sheet forming machine is available, among others, from Tendring Pacific Limited, United Kingdom, under the trade name Formax III.

An automatic pulp analyzing system is available from ABB Group under the trade name Lorentzen-Wettre. This system is capable of optical, NIR, CSF and gravimetrical analysis of a pulp suspension, i.e. it is only able to apply physical methods on pulp suspensions. No dissolution steps or application of other chemicals are possible with this system. A similar system is offered by PulpEye AB, Sweden.

However none of these systems offers the possibility for automatic measurement of the molecular mass of a polymer, e.g. cellulose.

Automatic viscometer systems are available e.g. from LAUDA DR. R. WOBSER GMBH & CO. KG/Germany. Besides the measurement itself they are capable of automatic filling and cleaning the Ubbelohde capillary viscometers which are generally used to determine the limiting viscosity number ("LVN") which allows the calculation of the molecular mass of a polymer. The same company offers autosampler systems which allow the preparation of a larger number of samples which are then analysed automatically. Nevertheless these systems require many steps which have to be done manually in advance to prepare the samples, like the taking of the samples from the plant, dosing and dissolving them and, depending on the analytics to be applied, even more sample preparation steps.

WO 2013/156147 A1 discloses an automatic online polymer analysis system as well as a corresponding method for continuously and automatically determining sticky levels in a process for producing recycled fiber pulp utilizing NIR spectroscopy. EP 0 700 719 A1 discloses an automatic measuring apparatus for several physical properties of cellulose derivative solutions. Amongst others the cellulose derivate solution is guided through a rotary viscometer.

### Problem

In view of this prior art the problem to be solved consisted in providing a system and a process for automated, integrated online supervising of the production process within a polymer manufacturing and processing plant. In case the polymer manufacturing and processing plant is a pulp plant and the pulp produced should be used in the manufacture of man-made cellulosic molded bodies the LVN is a crucial property of this product and has to be supervised thoroughly.

One of the problems of online analyses within polymer manufacturing and processing plants is that the fluid media may show inhomogeneities, in particular with regard to polymer concentration etc. Therefore a problem to be solved by the present invention is to ensure that the result of the analysis indeed represents the general conditions within the plant. Another problem to be solved is that the analytical results should be verified afterwards, if later on doubts about the correct results arise.

Yet another crucial problem of the analysis of suspended particles from a wet process by the LVN method is to obtain an exactly known amount of polymer for dissolving because the polymer concentration is a crucial parameter in calculating the LVN from the analytical data.

### Description

The usual approach to solve problems of this type is to develop a new analytical method that can be performed easily and reliably, mostly by indirectly measuring a physical parameter of the medium and establishing a correlation with parameters directly needed for the controlling of the manufacturing process in the plant. However developing such a method usually requires significant time and development cost and sometimes is impossible to find. The inventors of the present invention took another approach that surprisingly resulted in a reliable analytical system and process. This approach comprises an automation of actions on several different levels, i.e.:
a.) taking samples from several different locations in the plant,
b.) transformation of the sample into a form that is suitable for the subsequent analysis,
c.) the LVN analysis itself (capillary viscometry with metal-containing solvents like Cuoxam and Cuen is very critical with regard to impurities, atmospheric oxygen and residues in the viscometer)
and d.) controlling the plant based on the results of such analysis

It is therefore an object of the present invention to provide an automatic online polymer analysis system, suitable for controlling the manufacturing process in a polymer processing plant, containing:
a. A sampling system with two or more sampling locations located at different locations in the plant, suitable for taking fluid samples.
b. A multi-inlet sample collection unit with at least two sample inlets, which is controlled by the process control unit,
c. A sheet-forming unit,
d. A transfer device to transfer the sheet of step c. to the polymer analysis unit,
e. A fully automated polymer analysis unit and
f. A process control unit,
wherein the polymer analysis unit contains a fully automated sample-handling device and a fully automated solution viscosity measuring device. "Fully automated" means that the unit can operate for in principle unlimited time without intervention by humans, except for refilling the consumables and for unexpected events.

The sampling system contains various pipes from the sampling locations to the inlets of the multi-inlet sample collection unit and, as required, pumps or other devices that are suitable to move the fluid samples towards the analysis unit. "Fluid samples" means that the samples taken from the process can be either in liquid form or they consist of dissolved polymers in liquid solvent or of a flowable dispersion of solid particles dispersed in a liquid. One example of such a dispersion is a pulp made from a cellulosic raw material. The pulp usually consists of cellulosic fibers dispersed in an aqueous fluid, wherein the aqueous fluid may contain small or even significant amounts of other components like dissolved salts or other polymers like low molecular lignins, hemicelluloses, degradation products of cellulose and lignin and various other organic and inorganic components that are well known to the skilled in the art.

The multi-inlet sample collection unit may contain an appropriate number of inlets for the pipes of the sampling system. These inlets may be controlled by a corresponding number of valves, like e.g. ball valves, flap valves or a multi-inlet valve in a manner that allows only one open valve at the same time. Such kinds of arrangements may also be called "multiplexer".

Sheet-forming units are commercially available and widely used in particular in the paper industry; a suitable device is e.g. Valmet PEX. Such devices are usually used to evaluate physical parameters of the pulp, like whiteness degree and paper-forming capabilities. They were never used before for the measurement of molecular mass or LVN.

In a preferred embodiment of the invention the fully automated sample-handling device contains a conveyor, which transfers the sample to the sample insert station, and which also contains an integrated cutting device. The sample-handling device further allows determining the mass of the sample.

In a preferred embodiment of the invention the fully automated solution viscosity measuring device is a turn table unit with a rotatable support disc and a certain number of sample cups made of special steel: antimagnetic, not magnetizable, corrosion resistant, with PTFE inlet; each cup contains a magnetic stirring rod which always stays in the cup. The support disc is rotating in an arrangement with at least the following processing stations:
a. Sample inserting station, where the pulp sheet segment is stuffed into the sample cup by a suitable piston,
b. Dispersing station with magnetic stirrer drive,
c. Dissolving station with magnetic stirrer drive,
d. Automated capillary viscometer station with an automated cleaning and drying device; the capillary viscometer preferably is of the Ubbelohde type. The appropriate automation equipment is commercially available e.g. from Lauda Dr. R. Wobser GmbH, Lauda-Königshofen, Germany, but depending on the design of the fully automated solution viscosity measuring device, for the sake of integratibility, it may be recommendable to design it on one's own.
e. Cup cleaning station with magnetic stirrer drive, which is preferably equipped with a nozzle head for spraying cleaning liquid reaching into the cup; moreover, this cleaning station needs to be equipped with a proper method and device to exhaust the residual liquid, controlled by an algorithm which assures optimal synchronization of exhausting / flushing sequences.
f. Cup drying station.

Optionally the cup cleaning and the cup drying may be combined at one station.

The sample cups may be mounted on and moved by a linear holder instead of a round support disc, while the processing stations may be arranged linearly, as well. In another possible embodiment the sample cups are moved by a robot arm from one processing station to the subsequent one.

Instead of mounting the sample cups on a moving holder and having the processing stations at fixed locations, the sample cups may be mounted at fixed locations while the processing stations are moved from one cup to the other. However such embodiment would require a more complex logistics system for providing each station with the required materials (sample sheets, liquids, energy, vacuum etc..

In a further preferred embodiment of the invention the polymer analysis unit further contains:
- A thermostatic bath below the support disc; the bottom section of the cups is immersed in the thermostatic bath.
- A thermostat for the thermostatic bath, controlled in a way that avoids oscillation of the thermostat temperature. The way to control the actual temperature to be at all times within the specified range is given by the application of state of the art control loop design & controller tuning.

Preferably the fully automated polymer analysis unit is placed completely inside of a housing with controlled temperature. A suitable temperature may be 25°C.

Another object of the present invention is the use of an automatic online polymer analysis system as described above for controlling the manufacturing process in a polymer processing plant. In a possible and even preferred use according to the invention the polymer processing plant is a pulp mill and the polymer is cellulose. With appropriate amendments in the sample preparation, e.g. thorough washing and neutralizing e.g. in the sheet-forming unit, and sample-handling the system may also be used for the analysis of e.g. alkali cellulose before xanthogenation in a viscose fiber production process. The automatic online polymer analysis system according to the invention may be generally be used in manufacturing processes where the LVN is of relevance. Other possible applications are the analysis of the cellulosic raw material, e.g. pulp, in the manufacture of cellulosic manmade molded bodies, e.g. acetate, viscose, modal or lyocel fibers, or the process control in a paper mill or a biorefinery; the result of this analysis can be used directly to control the process parameters in the manufacturing process. In general, the invention may also be used in the analysis of other polymers, even synthetic thermoplastic polymers, provided that the sheet-forming unit is replaced by a film-forming unit, i.e. a device which forms a piece of polymer film of predetermined size. This film may be handled generally in the same way as the pulp sheet, however using an appropriate solvent etc..

It is another object of the present invention to provide a process for controlling the manufacturing process in a polymer processing plant, containing the sequence of the following steps:
a. Taking a fluid sample through a sampling system with two or more sampling locations,
b. Collecting the fluid sample by a multi-inlet sample collection unit with at least two sample inlets,
c. Forming a sheet by a sheet-forming unit,
d. Transferring the sheet to a fully automated polymer analysis unit,
e. Analyzing the polymer by the fully automated polymer analysis unit and
f. Controlling the manufacturing process by a process control system of the polymer processing plant using the data elaborated in steps a. to e..

The sampling system has at least one, mostly two or more sampling locations located at different locations in the plant, suitable for taking fluid samples. The number of sampling locations may be much higher than two. Three, four, five, six, seven, eight, nine, ten or even twenty sampling locations are possible, depending mostly on the requirements of the plant. However, at a given duration of one measurement the overall measurement rate achievable by the system according to the invention generally decreases with increasing number of sampling locations. At the sampling locations the polymer samples may be taken from the main stream by commercially available sample taking devices, e.g. working with moving pistons.

The fluid samples are transferred through pipes from the sampling locations to the inlets of the multi-inlet sample collection unit, using, if required, pumps or other devices or options which are suitable to move the fluid samples towards the analysis unit. Suitable pump types comprise peristaltic pumps, gear pumps, piston pumps, spiral pumps, mohno pumps, membrane pumps, centrifugal pumps, etc.. Transfer via a gas stream or liquid stream, such as e.g. an air stream or water stream, is possible as well. Fluid samples means that either the samples taken from the process can be in liquid form or they consist of dissolved polymers in liquid solvent or of a flowable dispersion of solid particles dispersed in a fluid. The present invention is in particular suitable for analyzing fluid samples wherein the dispersed solid particles are fibrous particles. One example of such a dispersion is a pulp made from a cellulosic raw material. The pulp usually consists of short cellulosic fibers dispersed in an aqueous fluid, wherein the aqueous fluid may contain small or even significant amounts of other components like dissolved salts or other polymers like low molecular lignins, hemicelluloses, degradation products of cellulose and lignin and various other organic and inorganic components which are well known to the skilled in the art.

The multi-inlet sample collection unit contains for example a controlled multi-inlet sampling valve or alternatively another type of multiplexer module, for instance made up of a series of individual one-way valves with at least one, but mostly two sample inlets and is controlled by the process control unit of the system according to the invention. The number of sample inlets may be much higher than two. Three, four, five, six, seven, eight, nine, ten or even twenty sample inlets are possible, depending mostly on the requirements of the plant. Usually it has as much sample inlets as sampling locations and the process control unit of the polymer analysis system controls from which sampling location a sample is taken and analyzed. This sample will then be transferred to the sheet-forming unit. Commercially available sheet-forming units usually contain a standard operation program to form a sheet of fibrous material from the incoming fluid sample.

Optionally the humidity of the sheets can be measured in order to further increase the exactness of the analysis. This may be done by generally known methods, either in the sheet-forming unit, or thereafter, but of course before dissolution of the cellulose, i.e. in or after step c. of the process according to the invention. The polymer analysis system according to the invention may contain a suitable measuring device.

To avoid any influence of inhomogeneity of the polymer content within the fluid sample, in a preferred embodiment of the present invention 3 sheets are formed from one fluid sample:
A first sheet is formed by the sheet-forming unit according to its standard procedure and the resulting area mass is evaluated automatically within the sheet-forming unit. The resulting data is used to enable the sheet-forming unit to form from the same fluid sample a second sheet that always shall have a specified target area mass. The reason is that from different fluid samples sheets with different area mass may be formed by the same sheet-forming unit, depending on e.g. the nature and geometric form of the polymer particles/fibers, the particle content in the sample, etc.. This second sheet will be used for the online analysis in the analysis unit.

A third sheet is formed from the same fluid sample. This sheet serves as a backup sample. It is transferred to a separate storage container, e.g. by a conveyor belt or other transfer device. This backup sample may be archived and analyzed by conventional methods whenever necessary, e.g. for validation of the results of the system according to the invention. This enables that the analytical results can be verified afterwards.

The individual charge number of the fluid sample is printed on each of the sheets by a sample coder.

The second sample sheet is further transferred to the fully automated polymer analysis unit by the fully automated sample-handling device. This fully automated sample-handling device contains a sample holder with integrated cutting device. The sample sheet is preferably transferred to the cutting device by a conveyor belt, while other devices capable of moving a sample sheet are generally suitable, too. The sample sheet is placed in the cutting device.

The blades of the cutting device should have a certain durability that fits to the automated operation mode of the whole system. E.g. if it is intended to operate the system for at least one day without any intervention by an operator, then the blades should have a service life time of much more than one day. Ideally, the service lifetime should be several years.

The cutting device cuts the sample sheet to a predetermined size. The resulting sample sheet segment must have a mass which is suitable for preparing a polymer solution for the viscometer in the polymer analysis unit and which is exactly known for the correct calculation of the LVN. This requires that the blades of the cutting device can be adjusted accordingly. Moreover the mass of each individual LVN sample must be determined with sufficient precision. For this purpose the sample sheet segment is transferred to the LVN-balance, weighted before being transferred into the appropriate sample cup at the sample inserting station of the fully automated solution viscosity measuring device. The transfer may be done by e.g. a pneumatically driven robot arm. The measured mass of the sample sheet segment is stored in the process control unit. The cup already contains a magnetic stirring rod.

The sample cup is then rotated to the subsequent dispersing station where the sample is dispersed in water by a magnetic stirrer to allow better dissolution in the subsequent step.

The sample cup containing the aqueous dispersion of the sample in water is then rotated to the subsequent dissolving station where a suitable solvent is added and the sample is dissolved. E.g. for measuring the molecular mass of cellulose there are various solvents known in the art. Generally preferred are the so-called Cuoxam, EWNN and Cuen. An applicable method for Cuen is described e.g. in ISO standard 5351/1-1981 (E). Of course, this description has to be combined with the specific operation description of the automated capillary viscometer used.

The sample cup containing the solution of the sample in the solvent is then rotated to the subsequent automated capillary viscometer station. There the viscosity is measured according to the program in the process control unit.

The results are stored in the process control unit. In the preferred embodiment of the invention, wherein the system is used to control a pulp mill, the essential result is the limiting viscosity number (usually given in the unit [ml/g]) or the degree of polymerization of the pulp sample. After the measurement the viscometer is automatically cleaned and dried. Commercially available automated capillary viscometers usually offer these functionalities and they can easily be integrated into the system according to the invention.

The sample cup containing the used solution of the sample is then rotated to the subsequent cup cleaning station. Here the cup is cleaned thoroughly. Suitable cleaning agents may be used. However in a preferred embodiment no other agents than demineralized water are used. The content of the cup is usually stirred with a magnetic stirrer to support the cleaning process. It is important for the performance of the whole system and method according to the invention that the cup is cleaned completely.

The cleaned, but still wet sample cup is then rotated to the subsequent cup drying station. The cup may be dried by an air stream or by any other suitable means. Optionally the cup can be dried in a separate step at a cup cleaning station. It is important for the performance of the whole system and method according to the invention that the cup is dried completely. The clean and dry cup will then be rotated to the sample inserting station to be filled with the next sample.

The system according to the invention contains a sample disc device with a support disc and a certain number of sample cups. This support disc is rotating in an arrangement of the processing stations as described above. Preferably the support disc contains either 5 or 6 cups, depending on whether the cleaning and the drying step are combined in one station or separated. Separated stations are preferred. Accordingly the system contains 5 or 6 processing stations and up to 5 or 6 samples are processed in the different stations of the system at the same time.

The process control unit has to be programmed accordingly in order to perform all processing steps at the same time, which allows a maximum of samples to be analyzed per time. Usually the procedure at the capillary viscometer station has the longest duration and therefore is the time-determining step. In the preferred use for a commercial-scale pulp manufacturing process a successful process control requires a measurement rate of at least 4 samples per hour.

For accurate results, it is crucial to perform the whole analysis at constant, well-defined temperature and air humidity. In a preferred embodiment of the invention the polymer analysis unit further contains a.) a thermostatic bath, filled with a liquid, below the rotating turn table, which contains the sample cup, the lower section of which is partly immersed in the thermostatic bath, and b.) a thermostat for the thermostatic bath, controlled in a way which avoids oscillation of the thermostat temperature. The way to control the actual temperature to be at all times within the specified range is given by the application of state of the art control loop design & controller tuning. The cups should be kept at controlled temperature at least at the dissolving station and the capillary viscometer station. Suitable thermostats are commercially available e.g. from LAUDA DR. R. WOBSER GMBH & CO. KG/Germany. Preferably the whole polymer analysis unit as well as the sample-handling device according to the invention are contained in a housing which is kept at predetermined, controlled temperature, even more preferred kept at controlled climate (temperature and air humidity), e.g. by air-condition. Preferably the complete housing containing the polymer analysis unit as well as the sample-handling device according to the invention is located in a room at controlled temperature or even at controlled climate.

All process steps in the whole system according to the invention as described above are controlled by a dedicated process control unit and therefore the process control unit has to be programmed accordingly. All physical operations in the system according to the invention are performed fully automatic by suitable electrical, mechanical or pneumatical actuators that are in principle known in the art. Furthermore all relevant operational data and results from the samples, among others the location where the sample was taken, its sampling time, internal identification number, all conditions of its analysis in the system according to the invention and the result of the viscosimetry are stored in the control unit of the system according to the invention. This data will also be transmitted directly and preferably without further interaction with a human operator, to the process control unit of the polymer processing plant and may be used therein to optimize the polymer processing conditions, e.g. the cooking process conditions in a wood pulp plant, e.g. by automatically influencing the addition of chemicals into the process.

Surprisingly it was found by the inventors that false analysis results caused by dirt, pollution in the liquids, insufficient cleaning etc. can be avoided by monitoring the pressure values in particular in the pipes of the capillary viscometer station and the cleaning station and to trigger alarm signals and other appropriate reactions, including appropriate signals to the process control system of the polymer processing plant, if predefined threshold values are reached or irregularities are detected. This specific control principle according to the invention also helps to increase the term of fully automated operation of the whole system without intervention of a human operator. Therefore in a preferred embodiment of the invention the pressure values in particular in the pipes of the capillary viscometer station and the cleaning station are monitored by the process control unit and alarm signals and other appropriate reactions, including appropriate signals to the process control system of the polymer processing plant are triggered, if predefined threshold values are reached or irregularities are detected.

Commercially available sheet-forming units usually furthermore contain a standard program to determine the degree of whiteness of the pulp sheets. This parameter can be used for controlling the polymer processing plant, too. It may e.g. serve as a product quality parameter.

The invention will now be illustrated by examples. These examples are not limiting the scope of the invention in any way. The invention includes also any other embodiments which are based on the same inventive concept.

### Examples

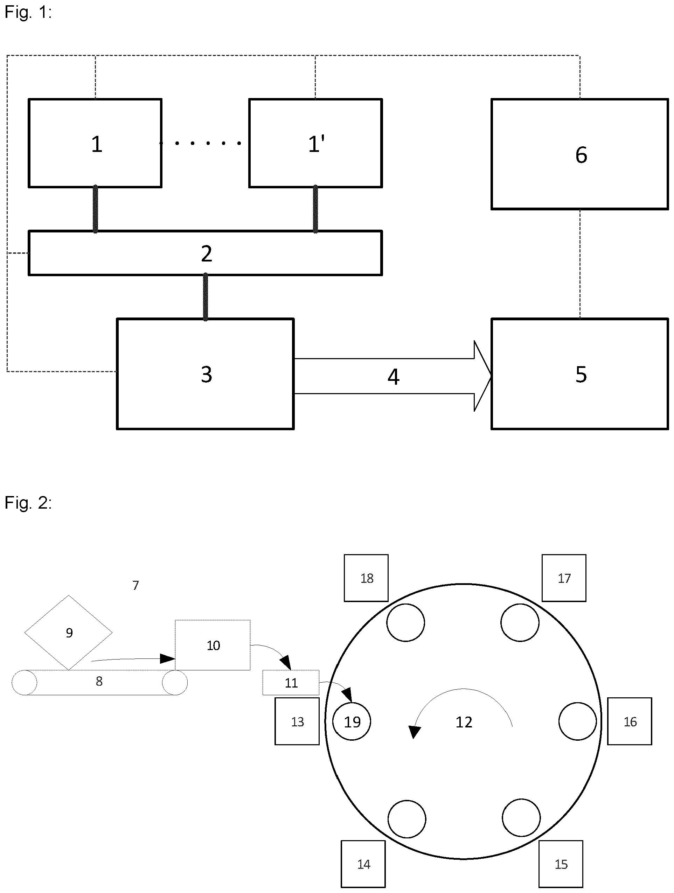

Fig. 1 and Fig. 2 show a preferred embodiment of the present invention. Fig. 1 shows a complete automatic online polymer analysis system. Fig. 2 shows a fully automated polymer analysis unit according to the invention.

Therein the individual parts are as follows, according to the description of the invention as outlined above:
- 1, 1':: Sampling locations; there may be more than two sampling locations in the system according to the invention
- 2:: Multi-inlet sample collection unit
- 3:: Sheet-forming unit
- 4:: Transfer device
- 5:: Fully automated polymer analysis unit
- 6:: Process control unit
- 7:: Sample-handling device
- 8:: Conveyor
- 9:: Sample coder
- 10:: Cutting device
- 11:: Balance
- 12:: Turn table; the arrow shows the direction of rotation in operation
- 13:: Sample inserting station
- 14:: Dispersing station with magnetic stirrer drive
- 15:: Dissolving station with magnetic stirrer drive
- 16:: Automated capillary viscometer station with an automated cleaning and drying device
- 17:: Cup cleaning station with magnetic stirrer drive
- 18:: Cup drying station
- 19:: One of the six cups mounted on the turntable

## Claims

1. Automatic online polymer analysis system, suitable for controlling the manufacturing process in a polymer processing plant, containing:
a. A sampling system with one or more sampling locations (1, 1'), suitable for taking fluid samples,
b. A multi-inlet sample collection unit (2) with a controlled multi-inlet sampling valve with at least two sample inlets,
c. A sheet-forming unit (3),
d. A transfer device (4),
e. A fully automated polymer analysis unit (5) and
f. A process control unit (6),
wherein the transfer device of the step d. is configured to transfer the sheet of step c. to the polymer analysis unit of step e.,
wherein the polymer analysis unit contains a fully automated sample-handling device (7) and a fully automated solution viscosity measuring device; **characterized in that** the fully automated solution viscosity measuring device is a turn table unit (12) with a rotatable support disc and a certain number of sample cups (19), rotating in an arrangement of at least the following processing stations:
g. Sample inserting station (13),
h. Dispersing station with magnetic stirrer drive (14),
i. Dissolving station with magnetic stirrer drive (15),
j. Automated capillary viscometer station with an automated cleaning and drying device (16),
k. Cup cleaning station with magnetic stirrer drive (17),
l. Cup drying station (18).

2. System according to claim 1, wherein the fully automated sample-handling device contains a conveyor belt and an integrated cutting device.

3. System according to claim 1, wherein the polymer analysis unit further contains:
• A thermostatic bath below the support disc,
• A thermostat for the thermostatic bath, controlled in a way which avoids oscillation of the thermostat temperature.

4. System according to claim 1, wherein the fully automated polymer analysis unit is placed completely inside of a housing with controlled temperature.

5. Use of an automatic online polymer analysis system according to claim 1 for controlling the manufacturing process in a polymer processing plant.

6. Use according to claim 6, wherein the polymer processing plant is a pulp mill and the polymer is cellulose.

7. Process for controlling the manufacturing process in a polymer processing plant by an online method, containing the sequence of the following steps:
a. Taking a fluid sample through a sampling system with one or more sampling locations (1, 1'),
b. Collecting the fluid sample by a multi-inlet sample collection unit (2) with one or more sample inlets,
c. Forming a sheet by a sheet-forming unit (3),
d. Transferring the sheet to a fully automated polymer analysis unit (5),
e. Analyzing the polymer by the fully automated polymer analysis unit (5), and
f. Controlling the manufacturing process by a manufacturing process control unit (6) of the polymer processing plant using the data elaborated in steps a. to e.,
**characterized in that** the polymer is analyzed by a fully automated solution viscosity measuring device that contains a turn table unit with a rotatable support disc and a certain number of sample cups (19), rotating in an arrangement of at least the following processing stations, executing the process steps:
g. Inserting the samples in the sample cup in a sample inserting station (13),
h. Dispersing the sample in a dispersing station with magnetic stirrer drive (14),
i. Dissolving the sample in a dissolving station with magnetic stirrer drive (15),
j. Measuring the solution viscosity in an automated capillary viscometer station with an automated cleaning and drying device (16),
k. Cleaning the sample cup in a cup cleaning station with magnetic stirrer drive (17),
l. Drying the sample cup in a cup drying station (18).

## Patentansprüche

1. Automatisches Online-Polymeranalyse-System, das für die Steuerung des Herstellungsprozesses in einer Polymerverarbeitungsanlage geeignet ist, enthaltend:
a. Ein Probenahmesystem mit einer oder mehreren Probenahmestellen (1, 1'), das zum Entnehmen von fluiden Proben geeignet ist,
b. Eine Probensammeleinheit (2) mit mehreren Eingängen und einem gesteuerten Probenahmeventil mit mehreren, mindestens aber zwei Probeneingängen,
c. Eine Blattbildungseinheit (3),
d. Eine Übergabevorrichtung (4),
e. Eine vollautomatisierte Polymeranalyseeinheit (5) und
f. Eine Prozesssteuerungseinheit (6),
wobei die Übergabevorrichtung des Schrittes d. dazu ausgestaltet ist, um das Blatt aus Schritt c. an die Polymeranalyseeinheit des Schrittes e. zu übergeben, wobei die Polymeranalyseeinheit eine vollautomatisierte Probenhandhabungsvorrichtung (7) und eine vollautomatisierte Lösungsviskositätsmessvorrichtung enthält, **dadurch kennzeichnet, dass** die vollautomatisierte Lösungsviskositätsmessvorrichtung aus einer Drehscheibeneinheit (12) mit einer drehbaren Trägerscheibe und einer bestimmten Anzahl von Probenbechern (19) besteht, die in einer Anordung von zumindest den folgenden Bearbeitungsstationen rotiert:
g. Probeneinfüllstation (13),
h. Dispergierstation mit Magnetrührerantrieb (14),
i. Auflösestation mit Magnetrührerantrieb (15),
j. Automatisierte Kapillarviskosimeterstation mit einer automatisierten Reinigungs- und Trocknungsvorrichtung (16),
k. Becherreinigungsstation mit Magnetrührerantrieb (17),
l. Bechertrockungsstation (18).

2. System gemäß Anspruch 1, wobei die vollautomatisierte Probenhandhabungsvorrichtung ein Transportband und eine integrierte Schneidvorrichtung enthält.

3. System gemäß Anspruch 1, wobei die Polymeranalyseeinheit weiterhin enthält:
• Ein Thermostatierbad unterhalb der Trägerscheibe,
• Einen Thermostat für das Thermostatierbad, der so gesteuert wird, das ein Oszillieren der Thermostattemperatur vermieden wird.

4. System gemäß Anspruch 1, wobei die vollautomatisierte Polymeranalyseeinheit vollständig innerhalb eines Gehäuses mit geregelter Temperatur untergebracht ist.

5. Verwendung eines automatischen Online-Polymeranalyse-Systems gemäß Anspruch 1 zur Steuerung des Herstellungsprozesses in einer Polymerverarbeitungsanlage.

6. Verwendung gemäß Anspruch 6, wobei die Polymerverarbeitungsanlage eine Zellstoffanlage ist und das Polymer Cellulose ist.

7. Verfahren zur Steuerung des Herstellungsprozesses in einer Polymerverarbeitungsanlage durch eine online-Methode, die die folgende Reihenfolge von Verfahrensschritten enthält:
a. Entnahme einer fluiden Probe mittels eines Probenahmesystems mit mit einer oder mehreren Probenahmestellen (1, 1'),
b. Sammeln der fluiden Probe mittels einer Probensammeleinheit (2) mit mehreren Eingängen mit einem oder mehreren Probeneingängen,
c. Bilden eines Blattes mittels einer Blattbildungseinheit (3)
d. Übergeben des Blattes an eine vollautomatisierte Polymeranalyseeinheit (5),
e. Analysieren des Polymers durch die vollautomatisierte Polymeranalyseeinheit (5), und
f. Steuern des Herstellungsprozesses durch eine Herstellungsprozesssteuerungseinheit (6) der Polymerverarbeitungsanlage unter Verwendung der Daten, die in den Schritten a. bis e. ermittelt worden waren,
**dadurch gekennzeichnet, dass** das Polymer durch eine vollautomatisierte Lösungsviskositätsmessvorrichtung analysiert wird, die eine Drehscheibeneinheit (12) mit einer drehbaren Trägerscheibe und einer bestimmten Anzahl von Probenbechern (19) enthält, die in einer Anordnung von zumindest den folgenden Bearbeitungsstationen rotiert und die folgenden Prozessschritte durchgeführt werden:
g. Einfüllen der Proben in die Probenbecher in einer Probeneinfüllstation (13),
h. Dispergieren der Probe in einer Dispergierstation mit Magnetrührerantrieb (14),
i. Auflösen der Probe in einer Auflösestation mit Magnetrührerantrieb (15),
j. Messen der Lösungsviskosität in einer automatisierten Kapillarviskosimeterstation mit einer automatisierten Reinigungs- und Trocknungsvorrichtung (16),
k. Reinigen des Bechers in einer Becherreinigungsstation mit Magnetrührerantrieb (17),
l. Trocknen des Bechers in einer Bechertrockungsstation (18).

## Revendications

1. Système d'analyse automatique d'un polymère en ligne, conçu pour contrôler le processus de fabrication dans une usine de transformation du polymère et comprenant :
a. Un système d'échantillonnage avec un ou plusieurs points d'échantillonnage (1, 1'), adapté au prélèvement d'échantillons fluides,
b. Une unité de collecte d'échantillons à entrées multiples (2) avec une vanne d'échantillonnage commandée à entrées multiples présentant au moins deux entrées d'échantillonnage,
c. Une unité de formation de feuilles (3),
d. Un dispositif de transfert (4),
e. Une unité d'analyse entièrement automatisée du polymère (5) et
f. Une unité de contrôle du processus (6),
le dispositif de transfert de l'étape d. étant configuré pour transférer la feuille de l'étape c. vers l'unité d'analyse du polymère de l'étape e., l'unité d'analyse du polymère comprenant un dispositif entièrement automatisé de manipulation d'échantillons (7) et un dispositif entièrement automatisé de mesure de la viscosité de la solution ;
**caractérisé en ce que** le dispositif entièrement automatisé de mesure de la viscosité de la solution est une unité à plateau tournant (12) avec un disque de support rotatif et un certain nombre de récipients à échantillons (19) tournant au centre d'un agencement comprenant au moins les postes de traitement suivants :
g. Poste d'insertion des échantillons (13),
h. Poste de dispersion avec entraînement pour agitateur magnétique (14),
i. Poste de dissolution avec entraînement pour agitateur magnétique (15),
j. Poste à viscosimètre capillaire automatisé avec un dispositif de nettoyage et de séchage automatisé (16),
k. Poste de nettoyage des récipients avec entraînement pour agitateur magnétique (17),
l. Poste de séchage des récipients (18).

2. Système selon la revendication 1, le dispositif entièrement automatisé de manipulation d'échantillons comprenant une bande de convoyage et un dispositif de coupe intégré.

3. Système selon la revendication 1, l'unité d'analyse du polymère comprenant en outre :
• Un bain thermostatique sous le disque de support,
• Un thermostat pour le bain thermostatique, commandé de manière à éviter toute oscillation de la température du thermostat.

4. Système selon la revendication 1, l'unité d'analyse entièrement automatisée du polymère étant entièrement placée à l'intérieur d'un logement à température contrôlée.

5. Utilisation d'un système d'analyse automatique d'un polymère en ligne selon la revendication 1 dans le but de contrôler le processus de fabrication dans une usine de transformation du polymère.

6. Utilisation selon la revendication 6, l'usine de transformation du polymère étant une usine de pâte et le polymère étant de la cellulose.

7. Processus de contrôle du processus de fabrication dans une usine de transformation du polymère par un procédé en ligne comprenant les étapes suivantes :
a. Prélèvement d'un échantillon fluide par un système d'échantillonnage avec un ou plusieurs points d'échantillonnage (1, 1'),
b. Collecte de l'échantillon fluide par une unité de collecte d'échantillons à entrées multiples (2) avec une ou plusieurs entrées d'échantillonnage,
c. Formation d'une feuille par une unité de formation de feuilles (3),
d. Transfert de la feuille vers une unité d'analyse entièrement automatisée du polymère (5),
e. Analyse du polymère par l'unité d'analyse entièrement automatisée du polymère (5), et
f. Contrôle du processus de fabrication par une unité de contrôle du processus de fabrication (6) de l'usine de transformation du polymère en utilisant les données produites aux étapes a. à e.,
**caractérisé en ce que** le polymère est analysé par un dispositif entièrement automatisé de mesure de la viscosité de la solution qui comprend une unité à plateau tournant avec un disque de support rotatif et un certain nombre de récipients à échantillons (19) tournant au centre d'un agencement comprenant au moins les postes de traitement qui exécutent les étapes suivantes du processus :
g. Insertion des échantillons dans le récipient à échantillons à un poste d'insertion des échantillons (13),
h. Dispersion de l'échantillon à un poste de dispersion avec entraînement pour agitateur magnétique (14),
i. Dissolution de l'échantillon à un poste de dissolution avec entraînement pour agitateur magnétique (15),
j. Mesure de la viscosité de la solution à un poste à viscosimètre capillaire automatisé avec un dispositif de nettoyage et de séchage automatisé (16),
k. Nettoyage du récipient à échantillons à un poste de nettoyage des récipients avec entraînement pour agitateur magnétique (17),
l. Séchage du récipient à échantillons à un poste de séchage des récipients (18).
